# EUROPEAN PATENT APPLICATION

(11) **EP 4 104 846 A1**
(43) Date of publication of application: **21.12.2022**
(21) Application number: 22178307.9
(22) Date of filing: 10.06.2022
(51) Int. Cl.: A61K 36/73, A61K 31/191, A61P 1/16, C12N 5/04

(54) **PREPARATION HAVING A PROTECTIVE ACTION TOWARDS HEPATIC FIBROSIS**

(30) Priority: 18.06.2021 IT 202100016028
(71) Applicant: ABresearch Srl, 36040 Brendola (VI) (IT)
(72) Inventor: DAL MONTE, Renzo, I-36040 BRENDOLA, VICENZA (IT); SACCANI, Andrea, I-36040 BRENDOLA, VICENZA (IT); BEDIN, Chiara, I-36040 BRENDOLA, VICENZA (IT)
(74) Representative: Croce, Valeria

(57) **Abstract**

It is an object of the present invention a preparation from a plant cell line for the protection against hepatic fibrosis.

## Description

### Technical field of the invention

The present invention finds application in the pharmaceutical, nutraceutical and medical fields, and in particular, relates to a protective preparation against hepatic fibrosis.

### Background art

Hepatic fibrosis is a disease causing the accumulation of connective tissue in the liver.

Often, hepatic fibrosis is the consequence of an injury already present in the liver tissue, caused by inflammatory lesions or other damage.

The continuous infiltration of fibrous tissue causes the progressive decrease of liver function.

Hepatic steatosis, on the other hand, is a pathological condition caused by the excessive accumulation of fat in the liver, and in particular, more than 5-10% of the weight of the organ.

There are different causes of hepatic steatosis ranging from genetic causes to environmental factors, such as obesity, diabetes or excess triglyceride levels, to behavioral factors, such as alcohol abuse and rapid weight changes.

Steatosis can cause inflammation of the liver, which is referred to as steatohepatitis.

The available pharmaceutical treatments can cause liver overload in a condition of impaired functionality.

The publication of Yilong et al, 2016 (DOI:10.3390/ijms17121983) describes an experiment on the hepatic effect of a phytocomplex comprising 37 different molecules, 22 of which are terpenoids, without any indication about the effect of the individual components.

International patent application WO 2020/097723 describes the preparation of an aqueous extract of apple peel, which thus comprises only hydrophilic (non-terpenoid) molecules, where the insoluble part is removed.

International patent application WO 2018/189672 describes the preparation of an aqueous extract, which thus comprises only hydrophilic (non-terpenoid) molecules of *Cynara cardunculus* and *Citrus aurantium.*

### Summary of the invention

The authors of the present invention have surprisingly found that a plant cell culture or an extract thereof is rich in triterpenes and has a protective action against hepatic fibrosis.

### Brief description of the drawings

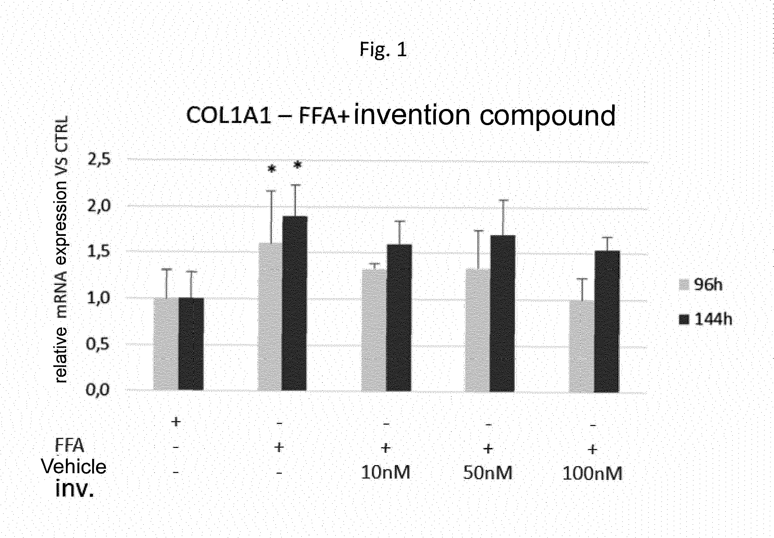

Figure 1 shows the results of COL1A1 gene expression after exposure to FFA and the compound of the invention ("inv.") after 96 and 144 hours. *p<0.05 vs CTRL.

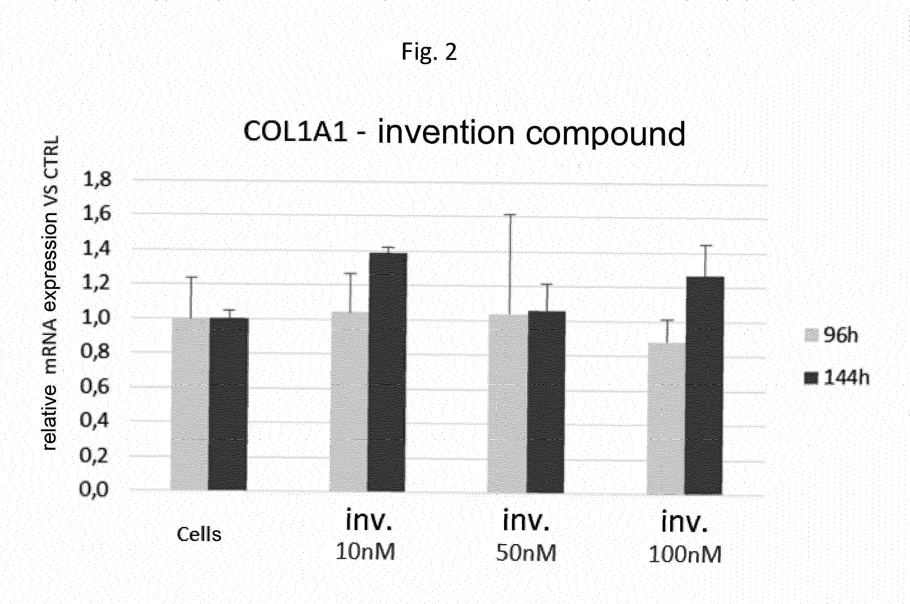

Figure 2 shows the results of COL1A1 gene expression to the compound of the invention ("inv.") in the absence of FFA after 96 and 144 hours. *p<0.05 vs CTRL.

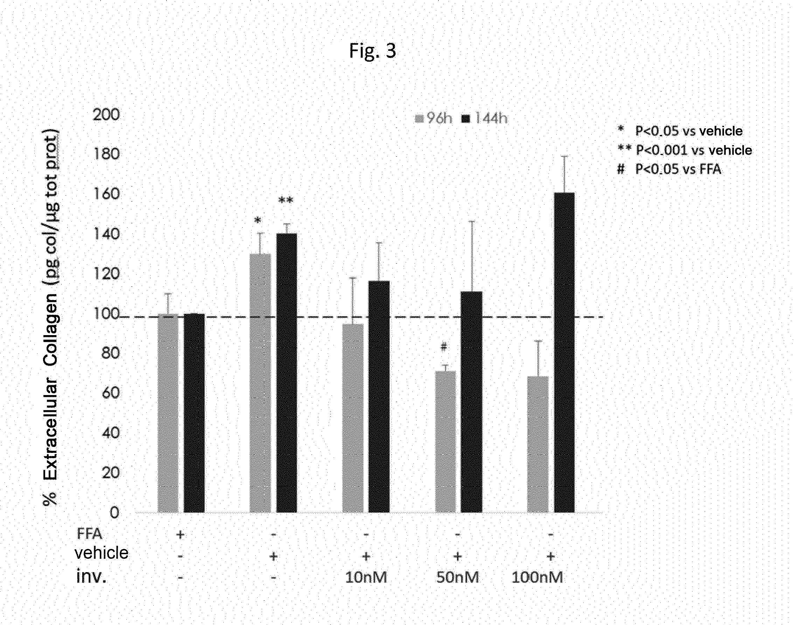

Figure 3 shows the results of the extracellular collagen quantification assays deposited after 96 hours (grey) and after 144 hours (black) of exposure to FFA and the compound of the invention ("inv.").

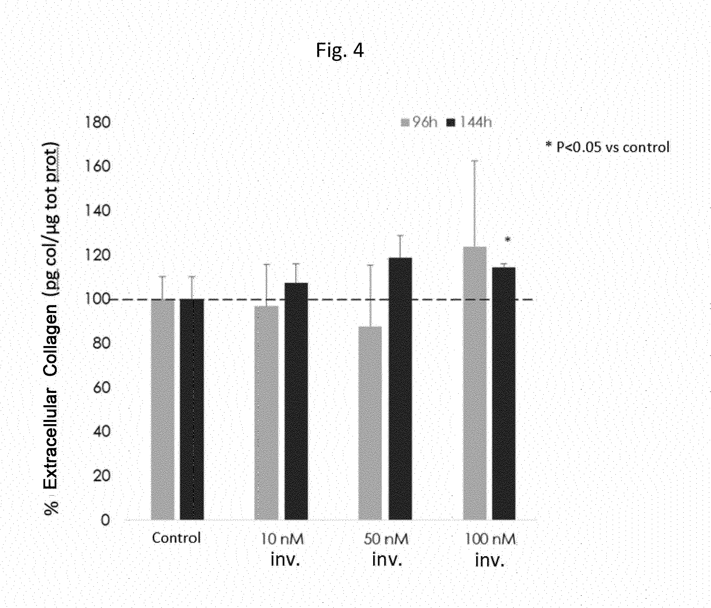

Figure 4 shows the results of the extracellular collagen quantification assays deposited after 96 hours (grey) and after 144 hours (black) of exposure to the compound of the invention ("inv."). *p<0.05 vs CTRL.

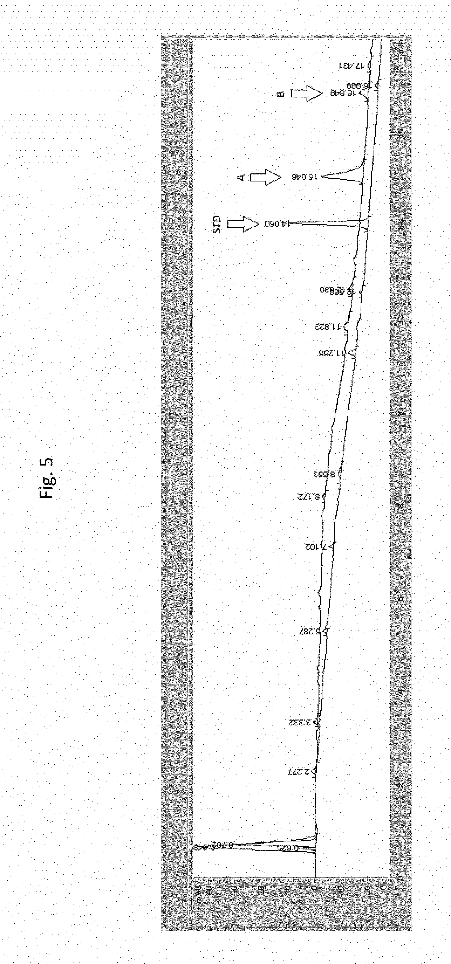

Figure 5 shows the HPLC chromatogram of the compound of the invention containing peak A (retention time: 15.046 minutes) and peak B (retention time: 16.849 minutes), superimposed on the HPLC chromatogram of tormentic acid standard (STD) at a known concentration of 53 µg/ml (retention time: 14.050 minutes).

### Object of the invention

According to a first object, the present invention describes a pharmaceutical, food and nutraceutical preparation comprising a cell culture, or an extract thereof, for medical use in the prevention and/or treatment of hepatic fibrosis.

For the purposes of the present invention, such a culture is a cell culture of a cell line.

In an aspect of the invention, such a preparation or an extract thereof is also described for medical use in the prevention and/or treatment of hepatic steatosis.

In a second object, there is described a formulation comprising the preparation of the invention or an extract thereof.

In a third object, there is described a method for protecting and/or treating hepatic fibrosis comprising the step of administering a preparation or an extract thereof according to the invention to a subject in need thereof.

In an aspect of the invention, such a method is for the prevention and/or treatment of hepatic steatosis as well.

In a further object, there are described euscaphic acid derivatives for medical use in the prevention and/or treatment of hepatic fibrosis and/or hepatic steatosis.

### Detailed description of the invention

According to a first object, the present invention describes a pharmaceutical, food or nutraceutical preparation comprising a cell culture, or an extract thereof, for medical use in the prevention and/or treatment of hepatic fibrosis.

The term "cell culture" means a culture developed in a fermenter from a specific and possibly selected cell line.

For the purposes of the present invention, the term "prevention of hepatic fibrosis" means the effect of preventing the onset of the disease in a subject at risk of developing such a disease for genetic or environmental (diet) predisposition reasons.

For the purposes of the present invention, the term "treatment of hepatic fibrosis" means the effect of regressing and/or healing the disease in a subject suffering therefrom.

The regression and/or healing are assessed with appropriate physiological or clinical parameters.

For the present purposes, such a preparation is a plant cell culture or an extract thereof.

In particular, such a plant cell culture is obtained from a fruit.

According to a particular aspect of the invention, such a fruit is the apple.

In an aspect of the invention, the plant cell culture or extract thereof is lyophilized.

In another aspect of the invention, the plant preparation can be a fraction of a fruit; such a fruit can be the apple.

According to a particularly preferred aspect of the present invention, the plant culture of apple fruit cells is characterized by a low sugar content as compared to a lyophilized preparation of the fruit.

More in detail, such a cell culture of apple fruit cells has a sugar content of less than 70% (w/w), preferably less than 60% (w/w), more preferably less than 50% (w/w) and even more preferably less than 40% (w/w).

According to another particularly preferred aspect of the invention, the cell culture of apple fruit cells is characterized by a high content of proteins and fibers.

More in detail, such a cell culture has a protein content 5 to 6 times higher with respect to the protein content of a lyophilized preparation of the fruit.

More in detail, such a cell culture has at least twice the fiber content of a lyophilized preparation of the fruit.

In particular, varieties of apples the cell lines of which can be used to obtain the preparation of the invention comprise the varieties: Granny Smith, Sibillini mountains pink apple, Rennet apple, quince, Golden Delicious apple, Fuji apple, Pinova apple, Golden PDO apple, Pink Lady Crisp apple.

To obtain the preparation of the invention, for example, plant tissue is first used, which is suitably sterilized and then reduced to small pieces (explants) for callogenesis.

With callogenesis, undifferentiated tissue develops, which is grown with appropriate replacement of the culture medium.

The process leads to the growth of a plant mass, which is finally collected, deprived of the culture liquid by filtration, washed and further filtered to then be subjected directly to lyophilization, or optionally, subjected to alcohol extraction and the extract subsequently lyophilized.

In an aspect of the invention, such a preparation is also described for medical use in the prevention and/or treatment of hepatic steatosis.

According to a second object of the invention, there is described a formulation comprising the preparation of the invention or an extract thereof.

Such a formulation can further comprise one or more pharmaceutically acceptable excipients.

For the purposes of the present invention, the preparation described or an extract thereof is rich in triterpenes.

In particular, such triterpenes comprise euscaphic acid (CAS 53155-25-2): 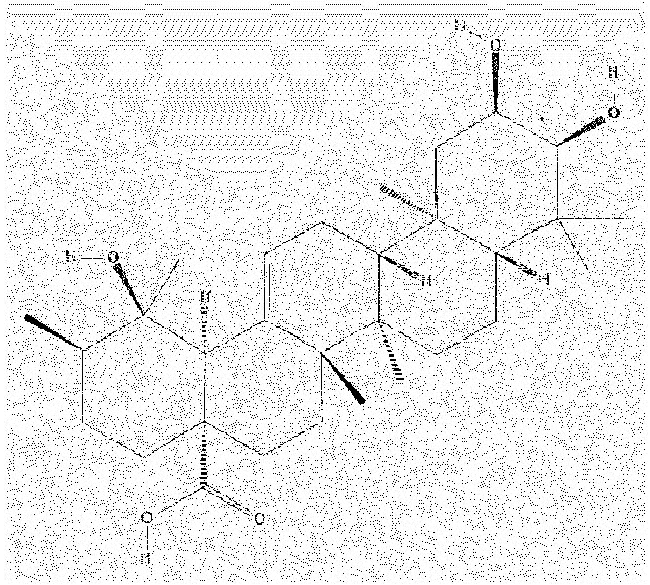 and/or derivatives thereof.

In particular, such derivatives are isomers of euscaphic acid.

In a preferred aspect of the invention, such derivatives are:
tormentic acid (CAS 13850-16-3): 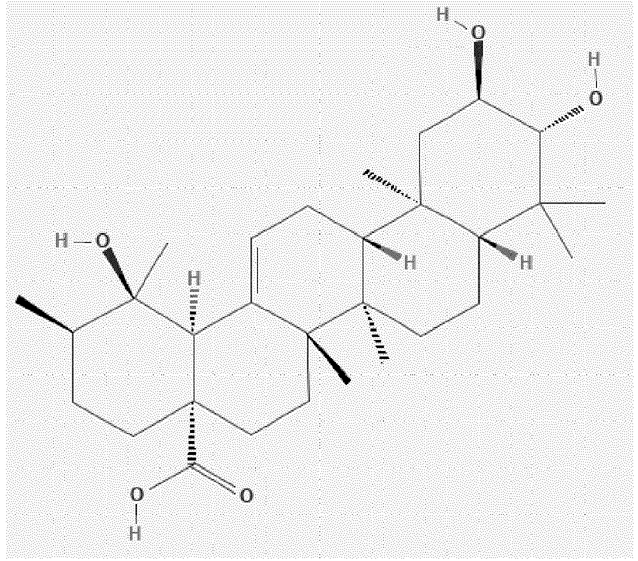
2-epi-tormentic acid (CAS 119725-19-8): 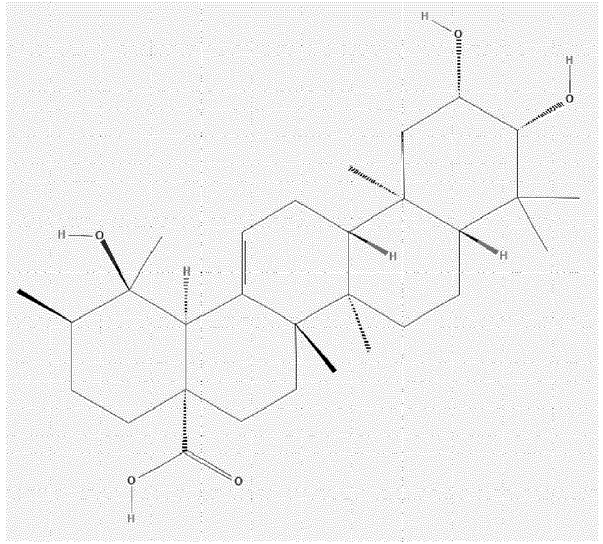
jacarandic acid (CAS 53155-25-2): 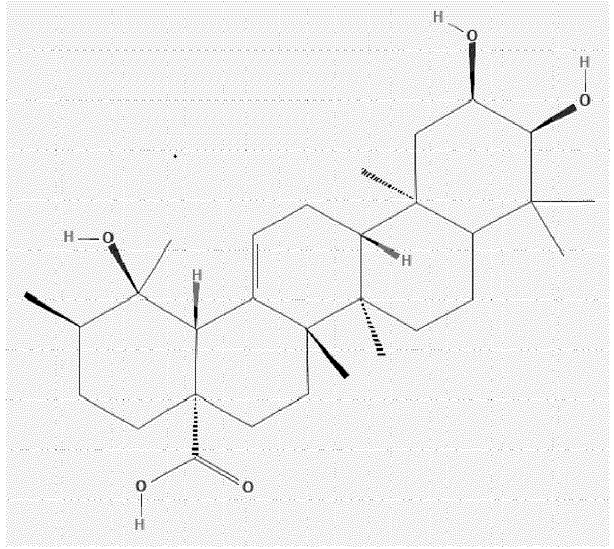 and 2R ,19R-dihydroxy-3-oxo-12-ursen-28-oic acid.

In an aspect of the invention, the disclosed preparation or an extract thereof does not comprise tormentic acid.

In a third object, there is also described a method for protecting and/or treating hepatic fibrosis, comprising the step of administering a preparation according to the invention or an extract thereof to a subject in need thereof.

In an aspect of the invention, there is also described a method for protecting and/or treating hepatic steatosis, comprising the step of administering a preparation according to the invention or an extract thereof to a subject in need thereof.

The present invention further describes the above-mentioned euscaphic acid derivative compounds for medical use in the prevention and/or treatment of hepatic fibrosis.

According to an aspect of the present invention, the above-mentioned euscaphic acid derivative compounds are also described for the prevention and/or treatment of hepatic steatosis.

The invention will be further described with reference to the results obtained and collected in the following tables.

### Methods

### Analysis by HPLC

The method developed includes HPLC-DAD separation by chromatography using the following reverse phase method: Chromolith^{®} RP-18e column (Merk), injection volume 0.5 ml, flow 1.2 ml/minute, temperature 25°C. The sample elution was performed by gradient 0-48% over 16 min to two eluents: A, consisting of 100% H₂O, B, consisting of 100% Acetonitrile.

The analyses reported the identification of 2 major peaks identified as A, with a retention time of 15.046 minutes and B, with a retention time of 16.849 minutes, which are not attributable to the peak of tormentic acid (standard) which has a peak with a retention time of 14.050 minutes.

### Analysis by nano-HPLC-Mass

The developed method includes the HPLC separation by gradient nanochromatography using a chip system (Chip-Cube, Agilent) containing precolumn (360 nL), column (750 nL) with C18 phase (Polaris) and tip for ESI-type ionization.

The chromatographic system consists of two pump systems: a system dedicated to pre-column loading and a second dedicated to column elution.

The loading was performed with eluent consisting of 50/50 methanol/water in the absence of acid.

The sample elution was performed by means of two-eluent gradient: A, consisting of H₂O, methanol, formic acid (97:3:0.1% v/v) B, consisting of acetonitrile, methanol, formic acid (60:40:0.1% v/v).

The gradient is performed by passing from the initial 50% of B to 90% in 20 minutes, after 4 minutes at 90%, 13 minutes of rebalancing to the initial phase follow. Flow 0.35 µl/minute; injection volume 0.2 - 0.5 µl

The conditions for mass spectrophotometry are as follows: negative voltage: 1600 V; fragmentor 160 V: desolvation air 4.5 L/min; temperature 325 degrees.

Sampling rate MS 3 spectra/sec, MS/MS 2 spectra/sec; fragmentation energy 40, 50, 60 eV

### Sample treatment.

The standard was diluted to obtain a 50% methanol solution containing 2 pMol/µl.

The samples were dried by speed-Vac and resuspended in 50% methanol.

The analyses led to the identification of the derivatives of tormentic acid: euscaphic acid, 2-epi-tormentic acid, jacarandic acid.

Euscaphic acid has been used in experimental tests to evaluate the activity thereof, as reported below.

### Materials and methods

### CELL CULTURE

Hepatocytes (HuH7) and HSC (LX2) were seeded together in a cellular ratio of 5: 1 (HuH7: LX2) which is the physiological cellular ratio and will form the SCC *(in vitro* simultaneous co-culture model). After the night, the cells were exposed to the preparation of the invention in the presence and absence of FFA (1200 µM). The experimental time for each condition was 96-144 hours.

In particular, the following was tested:
i. a control comprising only the vehicle
ii. a preparation of fatty acids (oleic and palmitic acid)
ii. preparation of the invention in the presence of the
   fatty acid preparation (oleic and palmitic acid)
The culture medium was refreshed every 48 hours. Vitality curves were performed at 96 and 144 hours in the presence or absence of FFA. After these exposure times, the cells were incubated for 1 hour with an MTT-containing medium at a concentration of 0.5 mg/ml. The medium was then removed, and the formazan crystals were dissolved in 200 µl of DMSO. 100 µl of each well was transferred to a microtiter plate and the optical density (OD) was determined at a wavelength of 562 nm.

### GENE EXPRESSION ANALYSIS

During this step, the gene expression was analyzed. Briefly, the total RNA was isolated using TRI-REAGENT (SIGMA) according to the manufacturer's instructions. The total RNA concentration was quantified spectrophotometrically at 260 nm in a Beckman Coulter DU^{®}730 spectrophotometer (Fullerton, CA, USA) and purity was evaluated by measuring the A260/A280 ratio. The total RNA (1 µg) was back-transcribed using high-capacity cDNA reverse transcription kits (Applied Biosystems, USA) according to the manufacturer's suggestions in a thermocycler (GeneAmp PCR System 2400, PerkinElmer, Boston, MA, USA). Quantitative PCR was performed in an iQ5 system (Bio-Rad, Hercules, CA USA) . The 18s and HPRT genes were used as reference. Amplification was performed in a 25 µL reaction volume containing 25 ng of cDNA, 1× iQ SYBR Green Supermix with 250 nM for the specific sense and antisense primers for the target genes, and 100 nM for the primers of the 18S gene.

### Alpha-LISA COL1A1 quantification

After 96 and 144 hours of treatments, the cells were lysed in AlphaLISA lysis buffer (Perkin Elmer, Waltham, MA USA) and stored at -80°C until use. The cell lysates were diluted 1:5 in Alpha Immunoassay buffer and the collagen content was quantified using the COL1A1 AlphaLISA Detection kit (Perkin Elmer, Waltham, MA USA) according to the manufacturer's instructions. The results were obtained by adapting the data to a dose-response sigmoidal curve generated with GraphPad prism^{®} version 5.01 and normalized to the total protein.

### Normalization vs total proteins

The total proteins were quantified by fluorescence, a non-fluorescent molecule which reacts readily with primary amines in amino acids and peptides to form stable, highly fluorescent compounds. 10 µl of fluorescamine at 4 mg/ml in DMSO was mixed with 40 µl of 1:2 diluted cell lysate. The fluorescence was read at 460 nm (excitation wavelength of 390 nm) in an EnSpire^{®} Multimode Plate Reader (Perkin Elmer, Waltham, MA USA) . The total protein was calculated using a dilution of the standard BSA curve.

### STATISTICAL ANALYSIS

The statistical analyses were performed using InStat software version 3.05 (GraphPad Software, Inc., La Jolla, CA, USA). The data are shown as mean ± SD of at least three independent biological replicates. The Student's t-test was performed for the statistical comparison of the groups. A p-value <0.05 was considered to be statistically significant.

### COL1A1 GENE EXPRESSION

FFA exposure causes time-dependent upregulation of collagen gene expression (COL1A1) with respect to CTRL (DMSO) (1.60 ± 0.56-fold; p = 0.04 at 96 hours and 1.89 ± 0.35-fold; p = 0.02 at 144 hours). A trend towards reduced COL1A1 expression was observed at both 96 hours and 144 hours.

### QUANTIFICATION OF EXTRACELLULAR COLLAGEN DEPOSITION

The 96-144 hour exposure to FFA (Free Fatty Acids) causes an increase in extracellular collagen deposit of 30 ± 10% (p <0.05) and 40 ± 5% (p <0.001), respectively, with respect to the vehicle-treated cells. The exposure showed a reduction in collagen after 96 hours for all experimental concentrations.

The experiments showed a reduction in COL1A1 gene expression (1.7-fold, p <0.05 vs FFA) and extracellular collagen deposition (50% p <0.05 vs FFA) at the lowest concentration tested.

## Claims

1. A pharmaceutical, food or nutraceutical preparation comprising a cell culture of a plant cell line or an extract thereof for the medical use in the prevention and/or treatment of hepatic fibrosis.

2. The pharmaceutical preparation according to the preceding claim, wherein the cell culture is an apple cell culture.

3. The pharmaceutical preparation according to the preceding claim comprising triterpenes.

4. The pharmaceutical preparation according to claim 1 or 2, wherein said triterpenes comprise: euscaphic acid, 2-epi-tormentic acid, jacaric acid, and 2R,19R-dihydroxy-3-oxo-12-ursen-28-oic acid.

5. The pharmaceutical preparation according to any one of the preceding claims, wherein said preparation is lyophilized.

6. The pharmaceutical preparation according to any one of the preceding claims 2 to 5, wherein said apple is an apple of a variety selected from the group comprising: Granny Smith, Marchigiana apple, Rennet apple, quince, Golden Delicious apple, Fuji apple, Pinova apple, Golden PDO apple, Pink Lady Crisp apple.

7. A formulation comprising the pharmaceutical preparation according to any one of the preceding claims, further comprising one or more pharmaceutically acceptable excipients.

8. The pharmaceutical preparation according to any one of the preceding claims 1 to 6, for the further medical use in the prevention and/or treatment of hepatic steatosis.

9. A derivative of tormentic acid selected from the group comprising: euscaphic acid, 2-epi-tormentic acid, jacaric acid, and 2R,19R-dihydroxy-3-oxo-12-ursen-28-oic acid for the medical use in the prevention and/or treatment of hepatic fibrosis.

10. A derivative of tormentic acid selected from the group comprising: euscaphic acid, 2-epi-tormentic acid, jacaric acid, and 2R,19R-dihydroxy-3-oxo-12-ursen-28-oic acid for the medical use in the prevention and/or treatment of hepatic steatosis.
